# FASCICULE DE BREVET EUROPEEN

(11) **EP 1 241 169 B1**
(45) Date de publication et mention de la délivrance du brevet: **27.08.2003**
(21) Numéro de dépôt: 02290598.8
(22) Date de dépôt: 11.03.2002
(51) Int. Cl.: C07D 487/04

(54) **Dérivés d'isoindoloindolone, leur procédé de préparation et les compositions pharmaceutiques qui les contiennent**
Isoindoloindolon-Derivate, Verfahren zu ihrer Herstellung und die enthaltende pharmazeutische Zusammensetzungen
Isoindoloindolone derivatives, process for their preparation and pharmaceutical compositions containing them

(30) Priorité: 12.03.2001 FR 0103293
(43) Date de publication de la demande: 18.09.2002
(73) Titulaire: Les Laboratoires Servier, 92200 Neuilly-sur-Seine (FR)
(72) Inventeur: Wierzbicki, Michel, 78620 l'Etang la Ville (FR); Boussard, Marie-Francoise, 78124 Mareil sur Mauldre (FR); Rousseau, Anne, 91160 Longjumeau (FR); Boutin, Jean-Albert, 92150 Suresnes (FR); Delagrange, Philippe, 92130 Issyles Moulineaux (FR)

(56) Documents cités:
- DATABASE CHEMABS [en ligne] CHEMICAL ABSTRACTS SERVICE, COLUMBUS, OHIO, US; BLACK, DAVID S. C. ET AL: "Synthesis of pyrrolophenanthridones by aryl-aryl coupling reactions" retrieved from STN Database accession no. 118:124844 XP002177413 & TETRAHEDRON (1993), 49(1), 151-64,

## Description

La présente invention concerne de nouveaux dérivés d'isoindoloindolones, leur procédé de préparation, les compositions pharmaceutiques qui les contiennent ainsi que leur utilisation dans le traitement des troubles du système mélatoninergique.

Quelques dérivés d'isoindoloindolones ont été décrits dans la littérature, notamment dans Tetrahedron 1993, 49 (1), 151-164, sans qu'aucune activité pharmacologique n'ait été décrite pour ces composés.

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272), analgésiques (Pharmacopsychiat., 1987, 20, pp. 222-223), ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p. 50 ; WO 97 04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une forte affinité pour les récepteurs de la mélatonine et une sélectivité importante pour les sites de type MT₃.

Plus particulièrement, la présente invention concerne les composés de formule (I): 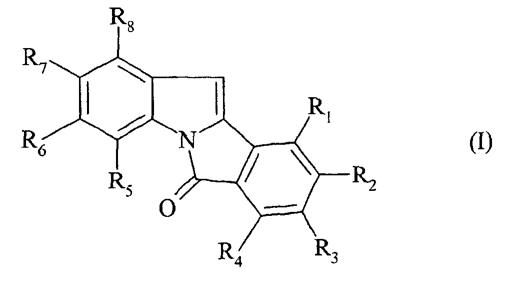 dans laquelle :
∗ R₁, R₂ R₃, R₄, R₅, R₆ et R₈, identiques ou différents, représentent chacun un atome d'hydrogène ou un groupement alkyle (C₁-C₆) linéaire ou ramifié, arylalkyle (C₁-C₆) linéaire ou ramifié, hydroxy, alkoxy (C₁-C₆) linéaire ou ramifié, arylalkoxy (C₁-C₆) linéaire ou ramifié, acyloxy (C₁-C₆) linéaire ou ramifié, arylcarbonyloxy, carboxyalkyle (C₁-C₆) linéaire ou ramifié ou carboxy,
∗ R₇ représente un atome d'hydrogène ou un groupement hydroxy, alkoxy (C₁-C₆) linéaire ou ramifié, arylalkoxy (C₁-C₆) linéaire ou ramifié, acyloxy (C₁-C₆) linéaire ou ramifié ou arylcarbonyloxy,
∗ ou bien l'un des groupements R₁ à R₈ forme avec un autre groupement R₁ à R₈ contigu un groupement alkylènedioxy (C₁-C₂),
leurs isomères optiques lorsqu'ils existent, ainsi que leurs sels d'addition à un acide ou à une base pharmaceutiquement acceptable,
à condition :
- que l'un au moins des groupements R₁ à R₈ représente un groupement hydroxy, alkoxy (C₁-C₆) linéaire ou ramifié, acyloxy (C₁-C₆) linéaire ou ramifié ou arylcarbonyloxy,
- et que les composés de formule (I) soient différents de la 1,3-diméthoxy-6H-isoindolo[2,1-a]indol-6-one.

Par groupement aryle, on entend phényle, biphényle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, amino (substitué éventuellement par un ou plusieurs groupements alkyle (C₁-C₆) linéaire ou ramifié), nitro, acyle (C₁-C₆) linéaire ou ramifié ou alkylènedioxy (C₁-C₂).

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, oxalique.

Parmi les bases pharmaceutiquement acceptables, on peut citer à titre non limitatif l'hydroxyde de sodium, l'hydroxyde de potassium, la triéthylamine, la tertbutylamine.

L'invention s'étend également au procédé de préparation des composés de formule (I) caractérisé en ce que l'on met en réaction un composé de formule (II) : 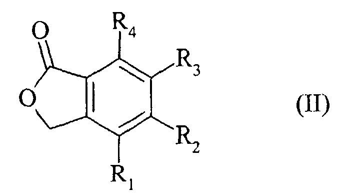 dans laquelle R₁, R₂, R₃ et R₄ ont la même signification que dans la formule (I),
avec le N-bromosuccinimide pour conduire au composé de formule (III) : 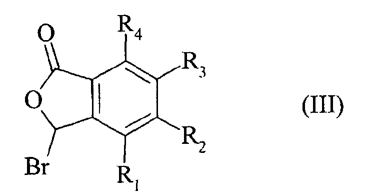 dans laquelle R₁, R₂, R₃ et R₄ ont la même signification que précédemment,
que l'on met en réaction avec la triphénylphosphine pour conduire au composé de formule (IV) : 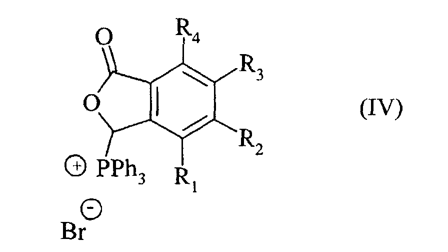 dans laquelle R₁, R₂, R₃ et R₄ ont la même signification que précédemment,
que l'on met en réaction avec un composé de formule (V) : 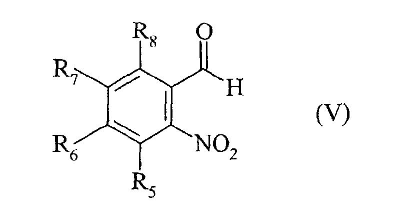 dans laquelle R₅, R₆, R₇ et R₈ ont la même signification que dans la formule (I),
pour conduire au composé de formule (VI) : 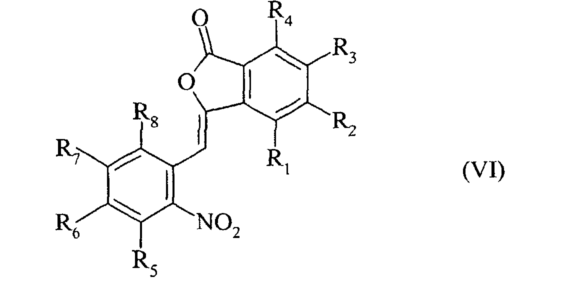 dans laquelle R₁, R₂, R₃, R₄, R₅, R₆, R₇ et R₈ ont la même signification que précédemment,
que l'on soumet à un agent de réduction pour conduire au composé de formule (VII) : 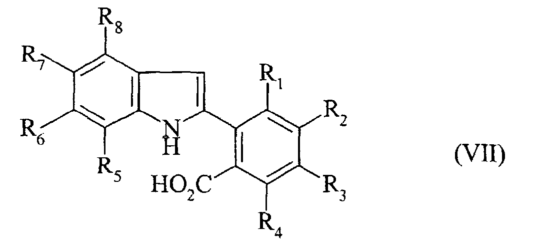 dans laquelle R₁, R₂, R₃, R₄, R₅, R₆, R₇ et R₈ ont la même signification qui précédemment,
que l'on cyclise ensuite pour conduire au composé de formule (I), que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide ou à une base pharmaceutiquement acceptable.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

### EXEMPLE 1 : 2-Hydroxy-8,9-diméthoxy-isoindolo[2,1-a]indol-6-one

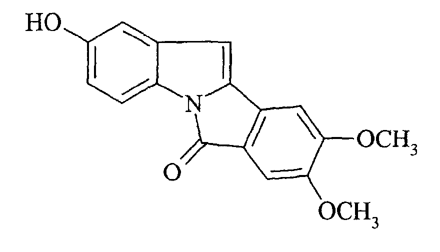

### Stade A : 3-Bromo-5,6-diméthoxy-phtalide

A 10 mmoles de 5,6-diméthoxy-phtalide en solution dans le dichlorométhane sont ajoutées 12 mmoles de N-bromo-succinimide, puis le mélange réactionnel, éclairé par une lampe halogène, est porté au reflux pendant 5 heures. Le mélange est ensuite ramené à température ambiante et filtré, puis le filtrat est évaporé, du toluène est ajouté, la suspension obtenue est filtrée et le filtrat est évaporé. Le résidu obtenu est filtré sur silice pour conduire au produit attendu.

### Stade B : Bromure de (5,6-diméthoxy-phtalidyl)-triphénylphosphonium

A 10 mmoles du composé obtenu dans le stade précédent en solution dans le toluène sont ajoutées 10 mmoles de triphénylphosphine, puis le mélange réactionnel est porté au reflux pendant 3 heures. Après retour à température ambiante, le mélange est filtré puis le gâteau obtenu est lavé et séché pour conduire au produit attendu.
*Point de fusion : > 260°C*

### Stade C : 3-(5-Hydroxy-2-nitro-benzylidène)-5,6-diméthoxy-phtalide

A 10 mmoles de 5-hydroxy-2-nitro-benzaldéhyde en solution dans le diméthylformamide sont ajoutées 10 mmoles de triéthylamine, puis, par portions, 10 mmoles du composé obtenu dans le stade précédent. Le mélange réactionnel est ensuite chauffé à 50°C pendant 1 heure 30, puis ramené à température ambiante et évaporé. De l'éther est ensuite ajouté, le mélange est agité pendant une nuit, puis filtré. Le gâteau obtenu est ensuite lavé pour conduire au produit attendu.
*Point de fusion* *: 253°C*

### Stade D : 3- (2 -Amino -5-hydroxy-benzylidène)-5,6-diméthoxy-phtalide

Une solution du composé décrit dans le stade précédent (10 mmoles) dans le diméthylformamide est placée sous hydrogène en présence de nickel de Raney jusqu'à absorption de 34 mmoles d'hydrogène. Après filtration du catalyseur, le solvant est évaporé et le résidu est séché pour conduire au produit attendu.
*Point de fusion : 231°C*

### Stade E : 2 - (2 -Carboxy-4,5-diméthoxy-phényl)-5-hydroxy-indole

A 10 mmoles du composé décrit dans le stade précédent en solution dans l'éthanol sont ajoutées 20 mmoles de soude en solution aqueuse 1N puis le mélange est porté au reflux pendant trois quarts d'heure. Après refroidissement à 0°C, le milieu est amené à pH = 1 à l'aide d'acide chlorhydrique 1N, puis après 1 heure à température ambiante, le précipité formé est filtré, lavé puis séché pour conduire au produit attendu.
*Point de fusion : 160°C*

### Stade F : 2-Hydroxy-8,9-diméthoxy-isoindolo[2,1-a]indol-6-one

Dans un ballon équipé d'un appareil de Dean-Stark sont introduites 10 mmoles du composé obtenu dans le stade précédent, en solution dans le toluène, puis 0,15 mmole d'acide paratoluènesulfonique. Après une nuit à reflux, le milieu réactionnel est ramené à température ambiante puis filtré. Le gâteau est ensuite lavé, puis du tétrahydrofurane est ajouté, la suspension est filtrée, le filtrat obtenu est évaporé et le résidu est lavé puis séché pour conduire au produit attendu.
*Point de fusion : > 260°C*

### Microanalyse élémentaire :

| | *C %* | *H%* | *N%* |
|---|---|---|---|
| *calculé* | *69,15* | *4,44* | *4,74* |
| *trouvé* | *69,05* | *4,18* | *4,98* |

### EXEMPLE 2 : 2-Hydroxy-10-méthoxy-isoindolo[2,1-a]indol-6-one

Le produit attendu est obtenu selon le procédé décrit dans l'Exemple 1 à partir de 4-méthoxy-phtalide et de 5-hydroxy-2-nitro-benzaldéhyde.
*Point de fusion :* 250°C

### Microanalyse élémentaire :

| | *C%* | *H%* | *N%* |
|---|---|---|---|
| *calculé* | *72,45* | *4,18* | *5,28* |
| *trouvé* | *72,14* | *4,31* | *5,28* |

### EXEMPLE 3 : 2-Hydroxy-7,10-diméthoxy-isoindolo[2,1-a]indol-6-one

Le produit attendu est obtenu selon le procédé décrit dans l'Exemple 1 à partir de 4,7-diméthoxy-phtalide et de 5-hydroxy-2-nitro-benzaldéhyde.
*Point de fusion :* > 260°C

### Microanalyse élémentaire :

| | *C%* | *H%* | *N%* |
|---|---|---|---|
| *calculé* | *69,15* | *4,44* | *4,74* |
| *trouvé* | *68,80* | *4,52* | *4,81* |

### EXEMPLE 4 : 2-Hydroxy-8-méthoxy-isoindolo[2,1-a]indol-6-one

Le produit attendu est obtenu selon le procédé décrit dans l'Exemple 1 à partir de 6-méthoxy-phtalide et de 5-hydroxy-2-nitro-benzaldéhyde.
*Point de fusion :* 217°C

### Microanalyse élémentaire :

| | *C%* | *H%* | *N%* |
|---|---|---|---|
| *calculé* | *72,45* | *4,18* | *5,28* |
| *trouvé* | *72,10* | *4,51* | *5,06* |

### EXEMPLE 5 : 2,9-Dihydroxy-8-méthoxy-isoindolo[2,1-a]indol-6-one

Le produit attendu est obtenu selon le procédé décrit dans l'Exemple 1 à partir de 5-hydroxy-6-méthoxy-phtalide et de 5-hydroxy-2-nitro-benzaldéhyde.
*Point de fusion : >* 260°C

### EXEMPLE 6 : 2,8-Dihydroxy-isoindolo[2,1-a]indol-6-one

Le produit attendu est obtenu selon le procédé décrit dans l'Exemple 1 à partir de 6-hydroxy-phtalide et de 5-hydroxy-2-nitro-benzaldéhyde.
*Point de fusion :* > 260°C

### EXEMPLE 7 : 8-Hydroxy-2-méthoxy-isoindolo[2,1-a]indol-6-one

Le produit attendu est obtenu selon le procédé décrit dans l'Exemple 1 à partir de 6-hydroxy-phtalide et de 5-méthoxy-2-nitro-benzaldéhyde.
*Point de fusion :* 110°C

### EXEMPLE 8 : 8-Hydroxy-2,3-méthylènedioxy-isoindolo[2,1-a]indol-6-one

Le produit attendu est obtenu selon le procédé décrit dans l'Exemple 1 à partir de 6-hydroxy-phtalide et de 4,5-méthylènedioxy-2-nitro-benzaldéhyde.
*Point de fusion :* 80°C

### EXEMPLE 9 : 2-Hydroxy-8,10-diméthoxy-isoindolo[2,1-a]indol-6-one

Le produit attendu est obtenu selon le procédé décrit dans l'Exemple 1 à partir de 4,6-diméthoxy-phtalide et de 5-hydroxy-2-nitro-benzaldéhyde.
*Point de fusion :* > 260°C

### Microanalyse élémentaire :

| | *C%* | *H%* | *N%* |
|---|---|---|---|
| *calculé* | *69,15* | *4,44* | *4*,*74* |
| *trouvé* | *69,06* | *4,42* | *4,77* |

### EXEMPLE 10 : 3-Benzyl-2-hydroxy-8,10-diméthoxy-isoindolo[2,1-a]indol-6-one

Le produit attendu est obtenu selon le procédé décrit dans l'Exemple 1 à partir de 4,6-diméthoxy-phtalide et de 4-benzyl-5-hydroxy-2-nitro-benzaldéhyde.

### EXEMPLE 11 : 2,7-Dihydroxy-isoindolo[2,1-a]indol-6-one

Le produit attendu est obtenu selon le procédé décrit dans l'Exemple 1 à partir de 7-hydroxy-phtalide et de 5-hydroxy-2-nitro-benzaldéhyde.
*Point de fusion :* 250°C

### EXEMPLE 12 : 2,7,10-Trihydroxy-isoindolo[2,1-a]indol-6-one

Le produit attendu est obtenu selon le procédé décrit dans l'Exemple 1 à partir de 4,7-dihydroxy-phtalide et de 5-hydroxy-2-nitro-benzaldéhyde.

### EXEMPLE 13 : 2,7-Dihydroxy-10-méthoxy-isoindolo[2,1-a]indol-6-one

Le produit attendu est obtenu selon le procédé décrit dans l'Exemple 1 à partir de 7-hydroxy-4-méthoxy-phtalide et de 5-hydroxy-2-nitro-benzaldéhyde.

### EXEMPLE 14 : 1-Carboxyéthyl-8,9-diméthoxy-isoindolo[2,1-a]indol-6-one

Le produit attendu est obtenu selon le procédé décrit dans l'Exemple 1 à partir de 5,6-diméthoxy-phtalide et de 6-carboxyéthyl-2-nitro-benzaldéhyde.

### EXEMPLE 15 : 8-Carboxy-7,9-diméthyl-2-hydroxy-isoindolo[2,1-a]indol-6-one

Le produit attendu est obtenu selon le procédé décrit dans l'Exemple 1 à partir de 6-carboxy-5,7-diméthyl-phtalide et de 5-hydroxy-2-nitro-benzaldéhyde.

### Etude pharmacologique des composés de l'invention

### EXEMPLE 16 : Etude de liaison aux sites de liaisons MT₃ de la mélatonine

La liaison sur les sites MT₃ est caractérisée par une cinétique d'association et de dissociation remarquablement rapide et par sa localisation tissulaire (cerveaux). Les expériences de liaison sur les sites MT₃ sont réalisées sur membranes de cerveau de hamster en utilisant la 2-[¹²⁵I] iodomélatonine comme radioligand suivant le protocole décrit par P. Paul et coll. (J. Pharmacol. Exp. Ther. 1999, 290, 334). Les membranes sont incubées pendant 30 minutes avec la 2-[¹²⁵I] iodomélatonine à la température de 4°C et différentes concentrations des composés à tester. Après l'incubation, les membranes sont rapidement filtrées puis lavées par du tampon froid à l'aide d'un système de filtration. La radioactivité fixée est mesurée par un compteur à scintillation.

Les valeurs d'IC₅₀ trouvées pour les composés de l'invention attestent d'une forte affinité pour les sites de type MT₃, ces valeurs étant inférieures à 10 nM. A titre de comparaison, la mélatonine présente une IC₅₀ de 45 nM dans ce test.

### EXEMPLE 17 : Composition pharmaceutique

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 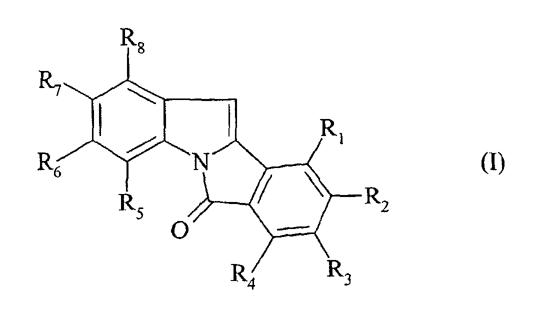 dans laquelle :
∗ R₁, R₂ R₃, R₄, R₅, R₆ et R₈, identiques ou différents, représentent chacun un atome d'hydrogène ou un groupement alkyle (C₁-C₆) linéaire ou ramifié, arylalkyle (C₁-C₆) linéaire ou ramifié, hydroxy, alkoxy (C₁-C₆) linéaire ou ramifié, arylalkoxy (C₁-C₆) linéaire ou ramifié, acyloxy (C₁-C₆) linéaire ou ramifié, arylcarbonyloxy, carboxyalkyle (C₁-C₆) linéaire ou ramifié ou carboxy,
∗ R₇ représente un atome d'hydrogène ou un groupement hydroxy, alkoxy (C₁-C₆) linéaire ou ramifié, arylalkoxy (C₁-C₆) linéaire ou ramifié, acyloxy (C₁-C₆) linéaire ou ramifié ou arylcarbonyloxy,
∗ ou bien l'un des groupements R₁ à R₈ forme avec un autre groupement R₁ à R₈ contigu un groupement alkylènedioxy (C₁-C₂),
ses isomères optiques lorsqu'ils existent, ainsi que ses sels d'addition à un acide ou à une base pharmaceutiquement acceptable,
à condition :
- que l'un au moins des groupements R₁ à R₈ représente un groupement hydroxy, alkoxy (C₁-C₆) linéaire ou ramifié, acyloxy (C₁-C₆) linéaire ou ramifié ou arylcarbonyloxy,
- et que les composés de formule (I) soient différents de la 1,3-diméthoxy-6*H*-isoindolo[2,1-a]indol-6-one.

2. Composé de formule (I) selon la revendication 1 qui est la 2-hydroxy-8,9-diméthoxy-isoindolo[2,1-a]indol-6-one.

3. Procédé de préparation des composés de formule (I) selon la revendication 1 **caractérisé en ce que** l'on met en réaction un composé de formule (II) : 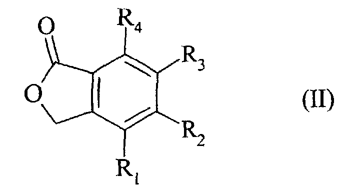 dans laquelle R₁, R₂, R₃ et R₄ ont la même signification que dans la formule (I),
avec le N-bromosuccinimide pour conduire au composé de formule (III) : 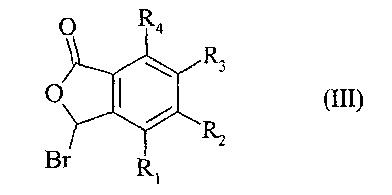 dans laquelle R₁, R₂, R₃ et R₄ ont la même signification que précédemment,
que l'on met en réaction avec la triphénylphosphine pour conduire au composé de formule (IV) : 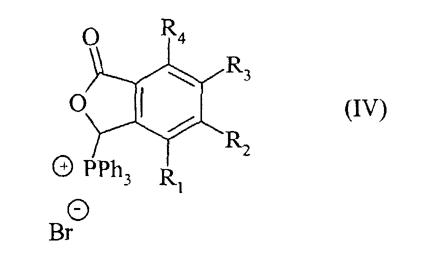 dans laquelle R₁, R₂, R₃ et R₄ ont la même signification que précédemment,
que l'on met en réaction avec un composé de formule (V) : 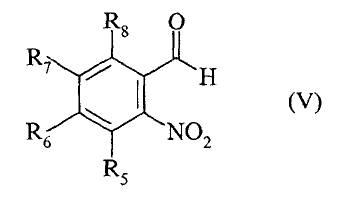 dans laquelle R₅, R₆, R₇ et R₈ ont la même signification que dans la formule (I),
pour conduire au composé de formule (VI) : 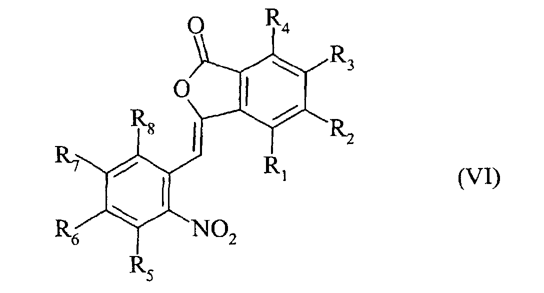 dans laquelle R₁, R₂, R₃, R₄, R₅, R₆, R₇ et R₈ ont la même signification que précédemment,
que l'on soumet à un agent de réduction pour conduire au composé de formule (VII) : 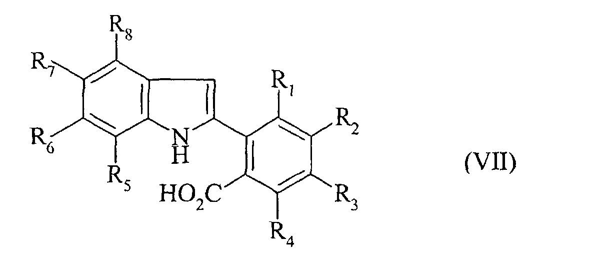 dans laquelle R₁, R₂, R₃, R₄, R₅, R₆, R₇ et R₈ ont la même signification que précédemment,
que l'on cyclise ensuite pour conduire au composé de formule (I), que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide ou à une base pharmaceutiquement acceptable.

4. Compositions pharmaceutiques contenant comme principe actif un composé de formule (I) selon l'une quelconque des revendications 1 ou 2, en combinaison avec un ou plusieurs véhicules inertes, non toxiques et pharmaceutiquement acceptables.

5. Compositions pharmaceutiques selon la revendication 4 utiles pour la fabrication de médicaments pour traiter les troubles du système mélatoninergique.

## Patentansprüche

1. Verbindung der Formel (I): 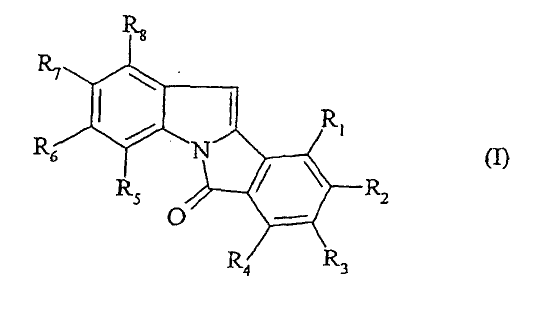 in welcher:
* R₁, R₂, R₃, R₄, R₅, R₆ und R₈, welche identisch oder unterschiedlich sind, jeweils ein Wasserstoffatom oder eine lineare oder verzweigte Alkylgruppe (C₁-C₆), lineare oder verzweigte Arylalkylgruppe (C₁-C₆), Hydroxygruppe, lineare oder verzweigte Alkoxygruppe (C₁-C₆), lineare oder verzweigte Arylalkoxygruppe (C₁-C₆), lineare oder verzweigte Acyloxygruppe (C₁-C₆), Arylcarbonyloxygruppe, lineare oder verzweigte Carboxyalkylgruppe (C₁-C₆) oder Carboxygruppe bedeuten,
* R₇ ein Wasserstoffatom oder eine Hydroxygruppe, lineare oder verzweigte Alkoxygruppe (C₁-C₆), lineare oder verzweigte Arylalkoxygruppe (C₁-C₆). lineare oder verzweigte Acyloxygruppe (C₁-C₆) oder Arylcarbonyloxygruppe bedeutet,
* oder eine der Gruppen R₁ bis R₈ mit einer anderen benachbarten Gruppe R₁ bis R₈ eine Alkylendioxygruppe (C₁-C₂) bildet,
deren optimische Isomere, falls diese existieren, sowie deren Additionssalze mit einer pharmazeutisch annehmbaren Säure oder Base, unter der Bedingung:
- dass mindestens eine der Gruppen R₁ bis R₈ eine Hydroxy-, lineare oder verzweigte Alkoxy- (C₁-C₆), lineare oder verzweigte Acyloxy- (C₁-C₆) oder Arylcarbonyloxygruppe ist,
- und dass die Verbindungen der Formel (I) verschieden von 1,3-Dimethoxy-6*H*-isoindolo[2,1-a]indol-6-on sind.

2. Verbindung der Formel (I) nach Anspruch 1, die 2-Hydroxy-8,9-dimethoxyisoindolo[2,1-a]indol-6-on ist.

3. Verfahren zur Herstellung der Verbindungen der Formel (I) nach Anspruch 1, **gekennzeichnet dadurch, dass** man eine Verbindung der Formel (II): 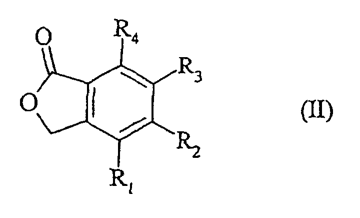 worin R₁, R₂, R₃ und R₄ dieselbe Bedeutung besitzen, wie in Formel (I),
mit N-Bromsuccinimid umsetzt, um eine Verbindung der Formel (III) zu erhalten 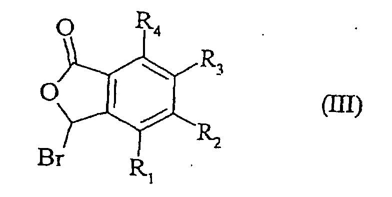 worin R₁, R₂, R₃ und R₄ dieselbe Bedeutung wie vorangehend besitzen,
dass man diese mit Triphenylphosphin umsetzt, um eine Verbindung der Formel (IV) zu erhalten: 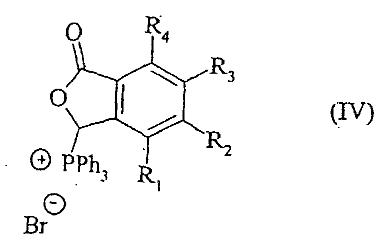 worin R₁, R₂, R₃ und R₄ dieselbe Bedeutung wie vorangehend besitzen,
dass man diese mit einer Verbindung der Formel (V): 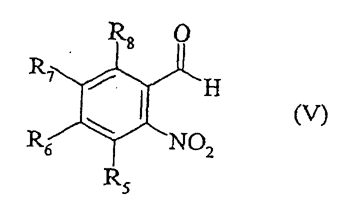 worin R₅, R₆, R₇ und R₈ dieselbe Bedeutung besitzen, wie in Formel (I),
umsetzt, um eine Verbindung der Formel (VI) zu erhalten: 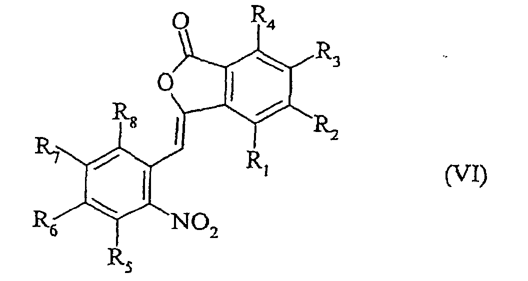 worin R₁, R₂, R₃, R₄, R₅, R₆, R₇ und R₈ dieselbe Bedeutung wie vorangehend besitzen,
dass man diese einem Reduktionsmittel unterwirft, um eine Verbindung der Formel (VII) zu erhalten: 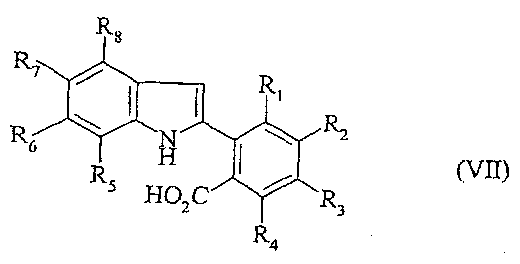 worin R₁, R₂, R₃, R₄, R₅, R₆, R₇ und R₈ dieselbe Bedeutung wie vorangehend besitzen,
dass man diese anschließend zyklisiert, um eine Verbindung der Formel (I) zu erhalten, diese, falls notwendig, gemäß einer klassischen Technik der Aufreinigung reinigt, die optischen Isomeren, falls gewünscht, gemäß einer klassischen Trennungstechnik trennt, und diese, falls gewünscht, in ihre Additionssalze mit einer pharmazeutisch annehmbaren Säure oder Base umwandelt.

4. Pharmazeutische Zusammensetzungen, welche als Wirkstoff eine Verbindung der Formel (I) nach irgendeinem der Ansprüche 1 oder 2, in Kombination mit einem oder mehreren inerten, nichttoxischen und pharmazeutisch annehmbaren Trägern, enthalten.

5. Pharmazeutische Zusammensetzungen nach Anspruch 4, zur Verwendung bei der Herstellung von Medikamenten zur Behandlung von Störungen des melatoninergen Systems.

## Claims

1. Compound of formula (I) : 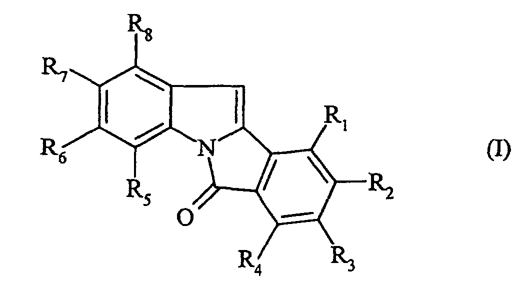 wherein :
* R₁, R₂ R₃, R₄, R₅, R₆ and R₈, which may be identical or different, each represents a hydrogen atom or a linear or branched (C₁-C₆)alkyl group, an aryl-(C₁-C₆)alkyl group in which alkyl may be linear or branched, a hydroxy group, a linear or branched (C₁-C₆)alkoxy group, an aryl-(C₁-C₆)alkoxy group in which alkoxy may be linear or branched, a linear or branched (C₁-C₆)acyloxy group, an arylcarbonyloxy group, a carboxy-(C₁-C₆)alkyl group in which alkyl may be linear or branched, or a carboxy group,
* R₇ represents a hydrogen atom or a hydroxy group, a linear or branched (C₁-C₆)alkoxy group, an aryl-(C₁-C₆)alkoxy group in which alkoxy may be linear or branched, a linear or branched (C₁-C₆)acyloxy group or an arylcarbonyloxy group,
* or one of the groups R₁ to R₈, together with another of the groups R₁ to R₈ adjacent to it, forms a (C₁-C₂)alkylenedioxy group,
optical isomers thereof, where they exist, and addition salts thereof with a pharmaceutically acceptable acid or base,
with the proviso:
- that at least one of the groups R₁ to R₈ represents a hydroxy, a linear or branched (C₁-C₆)alkoxy, a linear or branched (C₁-C₆)acyloxy or an arylcarbonyloxy group,
- and that the compounds of formula (I) are other than 1,3-dimethoxy-6*H*-isoindolo[2,1-a]indol-6-one.

2. Compound of formula (I) according to claim 1 that is 2-hydroxy-8,9-dimethoxy-isoindolo[2,1-a]indol-6-one.

3. Process for the preparation of compounds of formula (I) according to claim 1, **characterised in that** a compound of formula (II) : 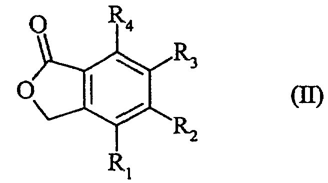 wherein R₁, R₂, R₃ and R₄ are as defined for formula (I),
is reacted with N-bromosuccinimide to yield a compound of formula (III) : 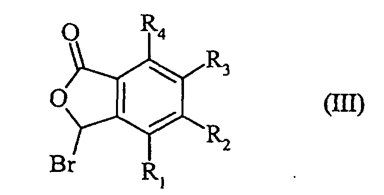 wherein R₁, R₂, R₃ and R₄ are as defined hereinbefore,
which is reacted with triphenylphosphine to yield a compound of formula (IV) : 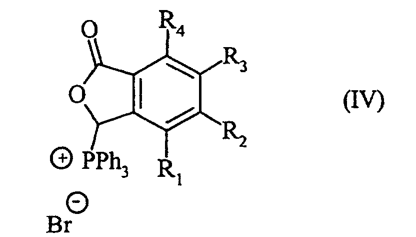 wherein R₁, R₂, R₃ and R₄ are as defined hereinbefore,
which is reacted with a compound of formula (V): 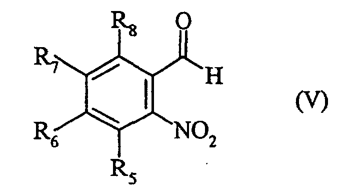 wherein R₅, R₆, R₇ and R₈ are as defined for formula (I),
to yield a compound of formula (VI) : 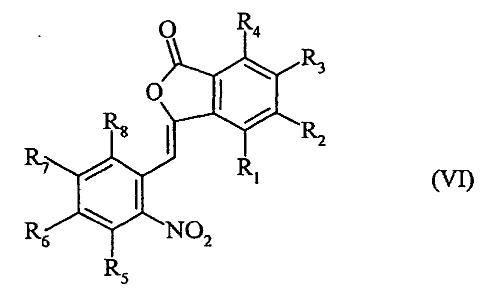 wherein R₁, R₂, R₃, R₄, R₅, R₆, R₇ and R₈ are as defined hereinbefore,
which is subjected to the action of a reducing agent to yield a compound of formula (VII) : 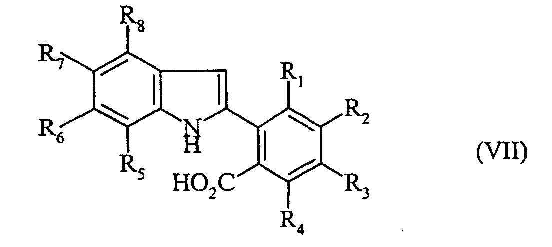 wherein R₁, R₂, R₃, R₄, R₅, R₆, R₇ and R₈ are as defined hereinbefore,
which is then cyclised to yield a compound of formula (I), which is purified, if necessary, according to a conventional purification technique, is separated, if desired, into its optical isomers according to a conventional separation technique and is converted, if desired, into addition salts with a pharmaceutically acceptable acid or base.

4. Pharmaceutical compositions comprising as active ingredient a compound according to either claim 1 or claim 2 in combination with one or more pharmaceutically acceptable, inert, non-toxic carriers.

5. Pharmaceutical compositions according to claim 4 for use in the preparation of medicaments for the treatment of disorders of the melatoninergic system.
